# EUROPÄISCHE PATENTSCHRIFT

(11) **EP 4 580 534 B1**
(45) Veröffentlichungstag und Bekanntmachung des Hinweises auf die Patenterteilung: **01.07.2026**
(21) Anmeldenummer: 24758788.4
(22) Anmeldetag: 20.08.2024
(51) Int. Cl.: A61B 18/14, A61B 17/29

(54) **SCHMETTERLINGSGRIFF EINES CHIRURGISCHEN HANDINSTRUMENTS**
BUTTERFLY GRIP OF A SURGICAL HANDHELD INSTRUMENT
POIGNÉE PAPILLON D'UN INSTRUMENT CHIRURGICAL PORTATIF

(30) Priorität: 22.08.2023 DE 102023122446
(43) Veröffentlichungstag der Anmeldung: 09.07.2025
(73) Patentinhaber: Aesculap AG, 78532 Tuttlingen (DE)
(72) Erfinder: BARTHELMES, Sven, 78576 Emmingen-Liptingen (DE); ROTHWEILER, Christoph, 78166 Donaueschingen (DE)
(74) Vertreter: Winter, Brandl - Partnerschaft mbB
(86) Internationale Anmeldenummer: PCT/EP2024/073340
(87) Internationale Veröffentlichungsnummer: WO 2025/040674

(56) Entgegenhaltungen:
- FR-A1- 2 688 681
- US-A- 5 201 743
- US-A1- 2009 299 141
- US-A1- 2013 150 665
- US-B1- 6 641 595

## Beschreibung

### Technisches Gebiet

Die Offenbarung betrifft den Handgriff eines oder für ein chirurgisches Instrument insbesondere elektrochirurgisches Instrument, sowie ein chirurgisches Instrument, insbesondere elektrochirurgisches Instrument mit einem Instrumentenschaft, zwei daran distal angeordneten zueinander zwischen einer Arbeitsstellung und einer Ruhestellung relativpositionierbaren / beweglichen Instrumentenbranchen und einem Handgriff, an dem zumindest ein manuell betätigbares Griff-/Bedienelement zur Relativpositionierung der Instrumentenbranchen zwischen einer Arbeitsstellung und einer Ruhe- bzw. Freigabestellung beweglich angeordnet ist. Beispiele für solche Instrumente sind Klemmen, Zangen und RF-Instrumente zur Verödung und/oder Koagulation von Gewebe.

### Hintergrund der Offenbarung

Es sind zum Beispiel chirurgische Instrumente bekannt, die mittels eines zangen- oder scherenartigen Werkzeuges/Instrumenteneffektors ein Greifen, Halten sowie Klemmen von Körpergewebe ermöglichen, um dieses dann durch Anlegen einer Hochfrequenzspannung an den Effektor monopolar oder bipolar zu koagulieren oder zu durchtrennen. Derartige Instrumente können allgemein als elektrochirurgische Instrumente bezeichnet werden. Klemmbackenartige Instrumentenbranchen eines solchen Werkzeugs/Instrumenteneffektors müssen insbesondere für einen Koagulationsvorgang in bestimmungsgemäßer Weise Druck auf das zwischen ihnen liegende Gewebe ausüben, um eine ausreichende Nahtfestigkeit zu gewährleisten, jedoch ein Durchschneiden des Gewebes zu verhindern. Zum Erreichen eines gewünschten Behandlungsergebnisses darf dieser Druck demzufolge weder zu groß (Zerstören von Gewebe) noch zu klein (unzureichendes Zusammenfügen von Gewebe) sein. D. h., insbesondere die beweglichen Griffelemente müssen so gestalten und angeordnet sein, dass eine möglichst genaue Dosierung der Betätigungskraft möglich ist. Gleiches gilt auch für jenen Betätigungskraft-Übertragungszug, der die auf die beweglichen Griffelemente aufgebrachte Betätigungskraft möglichst exakt auf den Instrumenteneffektor überträgt.

Elektrochirurgische Instrumente der einschlägigen Bauart verwenden in der Regel einen sogenannten Pistolenhandgriff mit einer starren/unbeweglichen Griffschale, an der ein Abzugsbügel oder -züngel schwenkbar angelenkt ist, der beispielsweise im Affengriff durch mehrere Finger der Greifhand gehalten und hin zur Griffschale manuell gezogen werden kann. Diese Ziehbewegung des Abzugsbügels wird über den Betätigungskraft-Übertragungszug innerhalb der starren Griffschale sowie innerhalb eines an den Pistolenhandgriff ankoppelbaren Instrumentenschafts auf das Werkzeug /Effektor des Instruments übertragen, um dieses entsprechend zu betätigen. Außerdem ist an dem Pistolenhandgriff eine Art Schalter angebracht, mittels dem die Strombeaufschlagung des Werkzeugs/Effektors ausgelöst werden kann.

Ein solcher herkömmlicher Pistolen-Handgriff, der ein feststehendes Griffteil und einen beweglichen Betätigungshebel aufweist, ist beispielsweise aus der US 2009/0299141 A1) bekannt.

Zudem ist ein herkömmlicher Scheren-Handgriff, der zwei diametral gegenüberliegende Griffbranchen aufweist, aus der US 5,201,743 A bekannt.

Eine andere Variante für einen Handgriff eines elektrochirurgischen Instruments ist der sogenannte Taschenlampenhandgriff, wonach ein im wesentlichen zylinderförmiges Gehäuse vorgesehen ist, an dessen äußerer Mantelseite ein Betätigungsbügel schwenkbar gelagert ist, der sich längs des zylinderförmigen Gehäuses erstreckt und über einen Betätigungskraft-Übertragungszug mit einem Werkzeug/Effektor am distalen Ende eines mit dem Handgriff gekoppelten Instrumentenschafts wirkverbunden ist.

Das Werkzeug/Effektor kann, wie vorstehend bereits angedeutet, beispielsweise aus zwei schwenkbar zueinander am Instrumentenschaft gelagerter (Gewebe-)Branchen bestehen, von denen zumindest eine über den Betätigungskraft-Übertragungszug bezüglich der anderen bewegbar, vorzugsweise schwenkbar ist, um dazwischen ein Patientengewebe greifend einzuspannen. Wenigstens eine der beiden Branchen kann mit einer Elektrode oder Elektrodenreihe ausgestattet sein, über die ein elektrischer Strom in das ergriffene Patientengewebe wahlweise eingeleitet werden kann. In diesem Fall läge ein elektrochirurgisches Instrument der monopolaren Bauart vor, bei der ein Patient beispielsweise auf einer Metallplatte aufliegt, über die der an der einen Branche eingeleitete elektrische Strom abgeleitet wird. Alternativ hierzu können aber auch beide, sich gegenüberliegenden (Gewebe-)Branchen des Effektors mit entsprechenden Elektroden ausgerüstet sein oder aus einem elektrisch leitfähigen Material bestehen, sodass der elektrische Strom nur noch in einem Spalt zwischen den Branchen fließt. In diesem Fall läge dann ein elektrochirurgisches Instrument der bipolaren Bauart vor.

### Stand der Technik

Aus der WO 2011/097469 A2 A1 ist ein chirurgisches HF-Instrument der Schaftbauart bekannt, mit zwei Gewebebranchen/Gewebeklemmelemente am distalen Ende des Instrumentenschafts, von denen eine Gewebebranche beweglich und die andere Gewebebranche feststehend ist. Der Instrumentenschaft ist an seinem proximalen Endabschnitt über eine Kupplungseinrichtung mit einem Instrumentenhandgriff der Pistolenhandgriffbauart gekoppelt und lässt sich durch die Kupplungseinrichtung relativ zum Handgriff um seine Längsachse drehen. Innerhalb des Instrumentenschafts ist eine Transmission in Form eines Zug-/Druckstabs gelagert, der distal an der einen beweglichen Gewebebranche angelenkt ist und proximal mit einem Getriebe innerhalb des Handgriffs gekoppelt/koppelbar ist. Am Handgriff ist schließlich ein Griffbügel (Betätigungsbranche) schwenkbar gelagert, der mit dem Getriebe wirkverbunden ist, um eine Schwenkwegung in eine Translationsbewegung des Zug-/Druckstabs zu überführen.

Aus der DE 10 2012 110 660 A1 ist ebenfalls ein chirurgisches HF-Instrument mit zwei Gewebebranchen/Gewebeklemmelementen bekannt, von denen zumindest eine relativ zur anderen bewegbar und über einen Betätigungsmechanismus (umfassend einen Betätigungsbügel und einen daran angekoppelten Betätigungskraft-Übertragungszug unter dazwischen Einklemmen von Körpergewebe mit einem vorbestimmten oder vorbestimmbaren Anpressdruck an die andere Gewebebranche anlegbar ist. Das Instrument weist zudem eine Klemmdruck-Regeleinrichtung auf, die in dem Kraft- oder Momentübertragungszug zwischen dem Betätigungsmechanismus und der zumindest einen bewegbaren Gewebebranche zwischengeschaltet und innerhalb des Griffgehäuses angeordnet ist. In diesem Fall handelt es sich bei dem Handgriff jedoch um einen zuvor allgemein umschriebenen Taschenlampenhandgriff mit einem einzigen bewegbaren Griffelement/ Betätigungsbügel/-hebel zur Bewegung einer der beiden Gewebebranchen.

Ein wichtiger Aspekt bei solchen Instrumenten ist deren Anwendungs- und Bedienfreundlichkeit.

Besonders zu nennen ist in diesem Zusammenhang die durch einen Anwender/Chirurgen bei einer Verwendung des Instruments aufzubringende Betätigungskraft. Die Instrumente sollen nämlich mit möglichst geringen Betätigungskräften präzise zu bedienen sein, um eine Ermüdung des Anwenders, in der Regel eines Operateurs/Chirurgen, sowie ggf. auftretende Nebeneffekte wie Zittern der das Instrument bedienenden Hand des Operateurs bei langem Halten oder relativ hohen Haltekräften gering zu halten. Des Weiteren sind geringe und für den Anwender vorausschaubare, also diesen nicht überraschende Betätigungskräfte förderlich für eine exakte, präzise und ruhige Handhabung des Instruments.

Schließlich spielt in der minimalinvasiven Chirurgie bzw. Endoskopie, insbesondere Laparoskopie, die Bauraum- und Gewichtsoptimierung der dort verwendeten chirurgischen Instrumente eine wichtige Rolle. So ist nicht nur bei dem distalen Werkzeug, das auch als Instrumentenkopf oder Effektor zu bezeichnen ist, sondern auch bei dem vom Anwender betätigten proximalen Instrumentenhandgriff auf eine platz- und gewichtssparende Ausgestaltung zu achten.

Weiterer Stand der Technik ist aus der DE 10 2019 107 197 A1, die ein Instrument mit einem Werkzeug und zwei als Ringbranchen ausgebildeten, zueinander verschwenkbaren Betätigungselementen zur Betätigung des Werkzeugs über ein Kopplungsmittel offenbart, aus der EP 4 054 447 B1, die ein Schiebeschaft-Instrument mit zwei zangenartig betätigbaren Betätigungselemente zur Verschiebung eines Schiebschafts offenbart, aus der US 5,954,731 A, die ein Instrument mit einem als Ganzes rotierbaren Handgriff, der ein feststehendes Griffteil und ein bewegliches Griffteil aufweist, offenbart, und aus der WO 2022/ 232 426 A1, die ein Instrument mit einem Handgriff offenbart, der über einen Schaft und einer darin gelagerten Transmission mit einem Endeffektor zu dessen Betätigung koppelbar ist, wobei der Schaft und die Transmission über eine Dreheinrichtung drehbar sind, bekannt.

### Kurzbeschreibung der Offenbarung

Die der Offenbarung zugrundeliegende Aufgabe ist, ein chirurgisches und insbesondere elektrochirurgisches Instrument sowie einen Handgriff eines oder für ein solches Instrument zu schaffen, das/der die vorstehend beschriebenen Eigenschaften verbessert, insbesondere einfach und mit geringen Betätigungs- und Haltekräften zu verwenden ist und/oder einfach aufgebaut ist, vorzugsweise mit geringem Gewicht und Bauraum.

Diese Aufgabe wird gelöst durch einen (medizinischen) Handgriff eines oder für ein chirurgisches Instrument insbesondere elektrochirurgisches Instrument gemäß den unabhängigen Patentansprüchen und/oder durch ein chirurgisches Instrument, insbesondere elektrochirurgisches Instrument gemäß dem nebengeordneten Patentanspruch. Vorteilhafte Ausgestaltungen und Weiterbildungen gemäß der Offenbarung sind Gegenstand der Unteransprüche, welche sämtlich unabhängig voneinander beanspruchbar sind.

Ein Kern der vorliegenden Offenbarung besteht folglich darin, das eingangs genannte Pistolenhandgriffprinzip zu verlassen und stattdessen auf ein sogenanntes _{"}Schmetterlingshandgriffprinzip" zu wechseln, wofür ein zentrales Griffgehäuse(-abschnitt) vorgesehen ist, in dem ein erster Teil eines Betätigungskraft-Übertragungszugs (der erste Teil wird nachfolgend als "Getriebe" bezeichnet) untergebracht ist und an seinem distalen Endabschnitt eine Kupplungseinrichtung zum Anschließen eines Instrumentenschafts samt darin gelagertem zweiten Teil des Betätigungskraft-Übertragungszugs (der zweite Teil wird nachfolgend als "Transmission" bezeichnet) angeordnet ist. Beidseits des zentralen Griffgehäuses sind (im Wesentlichen diametral zur Mittellinie des zentralen Griffgehäuses gegenüberliegend) jeweils ein Betätigungshebel/bewegliches Griffelement gelagert (also insgesamt zwei Betätigungshebel), die jeweils an ihren distalen Enden/Endabschnitten an einer Dreh-/Schwenkachse im/am zentralen Griffgehäuse gelagert sind, derart, dass die beiden Betätigungshebel aufeinander zu und voneinander weg verschwenkbar sind (vergleichbar zu den beiden Griffbranchen einer Schere oder Zange). Die beiden Betätigungshebel (bewegliche Griffelemente/Griffbranchen) erstrecken sich dabei längs des zentralen Griffgehäuses etwa in axial-paralleler Verlängerung zur Einkupplungsrichtung der Kupplungseinrichtung oder in einem spitzen Neigungswinkel zu dieser Einkupplungsrichtung sowie vorzugsweise über das proximale Ende des zentralen Griffgehäuses hinaus und bilden so quasi die Flügel des "Schmetterlings", dessen Torso wiederum quasi durch das zentrale Griffgehäuse dargestellt wird.

Weiter vorzugsweise sind die beiden sich gegenüberliegenden Dreh-/Schwenkachsen der bzw. für die beiden Betätigungshebel nicht achsparallel ausgerichtet, sondern deren Mittellinien sind in einem spitzen Winkel zueinander (V-förmig) angestellt. Des Weiteren definieren die beiden Mittellinien der Dreh-/Schwenkachsen eine Ebene, die im Bereich des Schnittpunkts mit der (distal-proximal ausgerichteten) Griffgehäusemittellinie im Wesentlichen senkrecht zu dieser Griffgehäusemittellinie ausgerichtet ist. Durch die spitzwinklige (V-förmige) Anstellung der beiden Dreh-/Schwenkachsen werden die Schwenkebenen der Betätigungshebel zueinander entsprechend diesem spitzen Winkel (zur Seite/quer zur distal-proximal-Richtung) gekippt (ähnlich des Flügelschlags eines Schmetterlings), um sich dadurch den beiden Schwenkebenen vom Daumen und den übrigen 4 Fingern einer menschlichen Hand im Falle einer Greif- und Freigabebewegung der menschlichen Hand anzupassen, bei der sich Daumen und Finger quasi auf einer Kreisbahn bewegen. Im Übrigen soll unter dem Begriff "im Wesentlichen senkrecht zu der Griffgehäusemittellinie" auch ein bezüglich der Griffgehäusemittellinie stumpfwinkliges/geringfügiges Abkippen der von den Mittellinien der Dreh-/Schwenkachsen aufgespannten Ebene in Richtung proximal (also nach hinten - unten) mitumfasst sein, derart, dass sich die Einkupplungsrichtung und die Längserstreckungsrichtung der beiden Betätigungshebel (in stumpfem Winkel) schneiden.

Demnach betrifft die Offenbarung einen Handgriff für ein chirurgisches Instrument, insbesondere ein elektrochirurgisches Instrument der minimalinvasiven Schaftbauart. Der Handgriff weist ein zentrales Griffgehäuse auf, das sich von distal in Richtung proximal erstreckt. Der Handgriff weist ein im zentralen Griffgehäuse aufgenommenes Getriebe auf. Der Handgriff weist eine kombinierte Kupplungs- und Dreheinrichtung am distalen Endabschnitt des zentralen Griffgehäuses auf. Die Kupplungs- und Dreheinrichtung ist zur wahlweisen Ankupplung sowohl eines Instrumentenschafts an das zentrale Griffgehäuse als auch einer im Instrumentenschaft gelagerten Transmission an das Getriebe vorgesehen und ausgebildet. Der Handgriff weist zwei diametral gegenüberliegend am Griffgehäuse positionierte Griffbranchen auf. Die Griffbranchen erstrecken sich beidseits des zentralen Griffgehäuses von distal in Richtung proximal. Die Griffbranchen weisen distale Endabschnitte auf, die am zentralen Griffgehäuse derart angelenkt sind, dass sie aufeinander zu und voneinander weg bewegbar sind. Die Griffbranchen weisen Kraftübertragungselemente oder Kraftübertragungsabschnitte auf, die jeweils an den distalen Endabschnitten ausgebildet oder angeordnet sind. Die Kraftübertragungselemente oder Kraftübertragungsabschnitte stehen mit dem Getriebe in Wirkeingriff stehen, um die Griffbranchenbewegung auf die Transmission zu übertragen.

Gemäß einer bevorzugten Ausführungsform kann der Handgriff zwei diametral gegenüberliegend am/im zentralen Griffgehäuse angeordnete Schwenkachsen haben, an denen die Griffbranchen angelenkt sind.

Gemäß einer bevorzugten Ausführungsform können die Kraftübertragungsabschnitte in Form von Aufnahmetaschen ausgebildet sind, welche in den distalen Endabschnitten der beiden Griffbranchen jeweils im Abstand zu den Schwenkachsen ausgeformt sowie einander zugewandt sind.

Gemäß einem ersten Aspekt der vorliegenden Offenbarung hat der Handgriff einen Querstab, der in Wirkeingriff mit den Kraftübertragungsabschnitten der beiden Griffbranchen steht.

Gemäß der bevorzugten Ausführungsform kann der Querstab an beiden Endabschnitten mit Kugeln oder Teilkugeln ausgebildet oder versehen sein, die in den Kraftübertragungsabschnitten, insbesondere in den beiden Aufnahmetaschen drehend sowie querstab-axial gleitend gelagert sind.

Gemäß der bevorzugten Ausführungsform kann der Handgriff einen wippenartig am/im zentralen Griffgehäuse gelagerten Hebel haben, der am Querstab angelenkt ist. An dem Hebel, insbesondere an dessen dem Querstab abgewandten freien Endabschnitt, kann eine Kupplungsaufnahme vorzugsweise in Form eines Schlitzes für die Transmission ausgebildet oder angeordnet sein.

Gemäß der bevorzugten Ausführungsform können/kann ein an dem Querstab angelenktes Ende des Hebels und/oder eine Querstabachse auf einer Kreisbahn um eine Wippenachse des Hebels beweglich sein. Eine Wippenachse des Hebels kann vorzugsweise senkrecht zu einer distal-proximal-Mittellinie und/oder zu einer Tiefenrichtung und/oder parallel zu einer Breitenrichtung sein. Die Tiefenrichtung und die Breitenrichtung des Handgriffs können vorzugsweise senkrecht zueinander sowie im Wesentlichen senkrecht zu der distal-proximal-Mittellinie des Handgriffs sein.

Gemäß einem zweiten Aspekt der vorliegenden Offenbarung sind die Kraftübertragungsabschnitte in Form von Zahnrädern oder Teilkreiszahnrädern ausgebildet, die mit einem Zentralzahnrad in Kämmeingriff stehen, dessen Drehachse parallel zu einer die Schwenkachsen verbindenden gedachten Linie ausgerichtet ist und welches eine Kupplungsaufnahme, vorzugsweise in Form eines Schlitzes, für die Transmission aufweist.

Gemäß der bevorzugten Ausführungsform können die Schwenkachsen in einem spitzen Winkel, vorzugsweise zwischen 0° und 25°, zueinander angestellt sein und zwar in einer Anstellebene, welche eine distal-proximal-Mittellinie des zentralen Griffgehäuses in einem Winkel kleiner-gleich 90°, vorzugsweise zwischen 90° und 65° schneidet.

Gemäß der bevorzugten Ausführungsform können eine Tiefenrichtung und eine Breitenrichtung des Handgriffs senkrecht zueinander sowie im Wesentlichen senkrecht zu einer distal-proximal-Mittellinie des Handgriffs sein. Gemäß der bevorzugten Ausführungsform können die Schwenkachsen jeweils, betrachtet in einer Ebene enthaltend die Tiefenrichtung und die Breitenrichtung, zur Tiefenrichtung im spitzen Winkel, insbesondere von 0° bis 25°, vorzugsweise von 5 bis 15°, geneigt sein. Gemäß der bevorzugten Ausführungsform können die Schwenkachsen jeweils, betrachtet in einer Ebene enthaltend die Tiefenrichtung und die distal-proximal-Mittellinie, zur Tiefenrichtung im spitzen Winkel, insbesondere von 0 bis 25°, vorzugsweise von 5 bis 20°, geneigt sein.

Gemäß der bevorzugten Ausführungsform können die distalen Endabschnitte jeder Griffbranche mit nach innen aufeinander zu ausgerichteten Vorsprüngen ausgebildet oder versehen sein. Die Vorsprünge können dafür vorgesehen und ausgebildet sein, als Aufschwenkbegrenzungen für die beiden Griffbranchen zu dienen, indem diese gegen den bereits angekuppelten Instrumentenschaft innerhalb des zentralen Griffgehäuses anschlagen.

Gemäß der bevorzugten Ausführungsform kann beim Abkuppeln des Instrumentenschafts vom Handgriff die Aufschwenkbegrenzung entfallen und damit ein zusätzliches Aufschwenken der beiden Griffbranchen in eine Einkupplungsschwenkposition erlaubt sein, in der die Transmission mit der Kupplungsaufnahme, vorzugsweise in Form eines Schlitzes, in und außer Eingriff bringbar ist.

Gemäß der bevorzugten Ausführungsform können die Kraftübertragungsabschnitte in Tiefenrichtung zumindest einseitig offen, vorzugsweise zu einer Handgriffoberseite hin, offen ausgebildet sein.

Gemäß der bevorzugten Ausführungsform können die Griffbranchen jeweils eine Fingeröse aufweisen. Die Fingeröse können an ihren jeweiligen proximalen Ösenabschnitten umfangsseitig offen ausgebildet sein, insbesondere im Wesentlichen eine nach proximal geöffnete U-Form aufweisen.

Gemäß der bevorzugten Ausführungsform kann die Kupplungs- und Dreheinrichtung einen Schaft-Kupplungsabschnitt zur wahlweisen Ankupplung, insbesondere axial/translationsfesten und drehbaren Aufnahme, des Instrumentenschafts an dem Griffgehäuse und einen Transmissions-Kupplungsabschnitt zur wahlweisen Ankupplung, insbesondere axial/translationsfesten Aufnahme, der Transmission an das Getriebe aufweisen.

Gemäß einer bevorzugten Ausführungsform können die Griffbranchen jeweils zu einer anderen Griffbranche der zwei Griffbranchen schwenkbar an dem Griffgehäuse angelenkt sein. Das Getriebe kann einen mit den Griffbranchen gekoppelten oder koppelbaren Griffbranchen-Kupplungsabschnitt aufweisen und ausgebildet sein, um, insbesondere im an den Handgriff gekoppelten Zustand des Instrumentenschafts und der Transmission, eine Schwenkbewegung der Griffbranchen in eine Translationsbewegung der Transmission zu übertragen.

Die Offenbarung betrifft auch ein chirurgisches Instrument, insbesondere elektrochirurgisches Instrument der minimalinvasiven Schaftbauart. Das Instrument weist einen Instrumentenschaft auf. Das Instrument weist eine Transmission, vorzugsweise in Form eines Zug-Druckstabs, auf. Die Transmission ist in dem Instrumentenschaft gelagert. Der Instrumentenschaft weist einen distalen Endabschnitt auf. Das Instrument weist einen Endeffektor auf, der an dem distalen Endabschnitt des Instrumentenschafts angeordnet ist. Der Endeffektor ist über die Transmission mechanisch betätigbar und ggf. über elektrische Leiter mit einem elektrischen Strom beaufschlagbar.

Das Instrument weist den beschriebenen Handgriff auf. Dabei sind der Instrumentenschaft und die darin gelagerte Transmission mit dem Handgriff gekoppelt oder koppelbar ist, insbesondere der Instrumentenschaft mit dem zentralen Griffgehäuse und die Transmission mit dem im zentralen Griffgehäuse untergebrachten Getriebe gekoppelt oder koppelbar.

### Kurzbeschreibung der Figuren

Die Offenbarung wird nachstehend anhand bevorzugter Ausführungsbeispiele unter Bezugnahme auf die begleitenden Figuren näher erläutert.
Fig. 1a, 1b zeigen die Perspektivenansicht eines Handgriffs für ein oder eines elektrochirurgischen Instruments mit Instrumentenschaft in ausgekuppeltem und eingekuppeltem Zustand gemäß der vorliegenden Offenbarung,
Fig. 2 zeigt einen ersten Teil eines Betätigungskraft-Übertragungszugs (nachfolgend als "Getriebe" bezeichnet) innerhalb eines feststehenden Griffteils (nachfolgend als "zentrales Griffgehäuse" bezeichnet) gemäß einem ersten bevorzugten Ausführungsbeispiel der Offenbarung und
Fig. 3 zeigt einen ersten Teil eines Betätigungskraft-Übertragungszugs innerhalb des nur in Fig. 2 dargestellten feststehenden Griffteils gemäß einem zweiten bevorzugten Ausführungsbeispiel der Offenbarung.

### Figurenbeschreibung

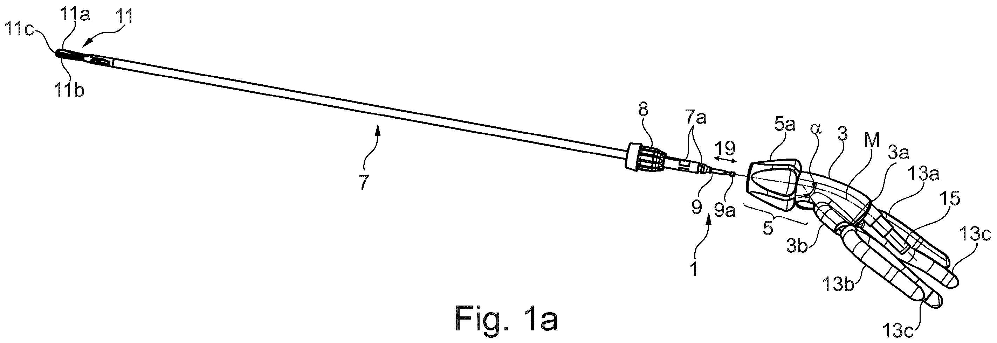

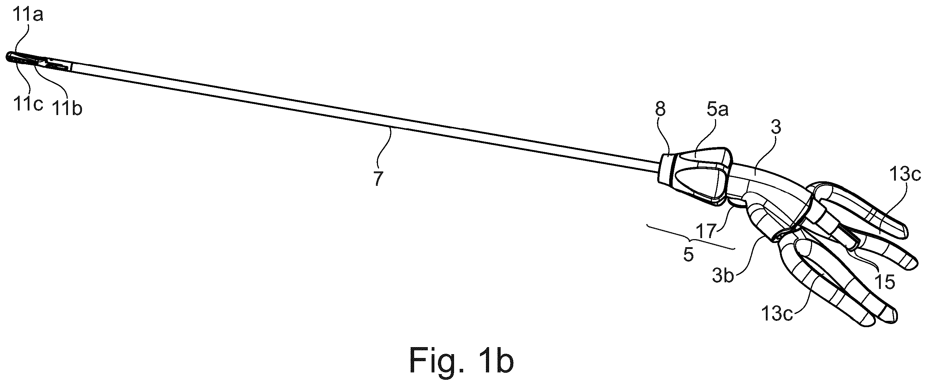

Der in den Fig. 1a, 1b dargestellte offenbarungsgemäße Handgriff 1 für ein oder eines chirurgischen, insbesondere elektrochirurgischen Instruments hat im Wesentlichen
- ein feststehendes Griffteil (zentrales Griffgehäuse) 3,
- eine manuell betätigbare Kupplungs- und Dreheinrichtung 5 zur Ankupplung eines in Fig. 1 nur angedeuteten Instrumentenschafts 7 mit entsprechender Schaftkupplung 7a und einer darin gelagerten Transmission 9 mit entsprechender Tranmissionskupplung 9a an den Handgriff 1, sowie zum Drehen des Instrumentenschafts 7 bezüglich des Handgriffs 1 mittels eines Drehknopfs 5a, der mit einem drehfest am Instrumentenschaft 7 gehaltenen Schafteingriffsstück 8 in Wirkeingriff bringbar ist, wobei mit Bezug auf den konstruktiven Aufbau und die Funktion der Kupplungs- und Dreheinrichtung 5 sowie des Instrumentenschafts 7 mit distalem Werkzeug/Effektor 11 beispielsweise auf die WO 2011/097469 A2 A1 verwiesen werden kann,
- zwei bewegliche Griffelemente/Griffbranchen 13a, 13b, die auf zwei voneinander abgewandten (also sich im Wesentlichen diametral gegenüberliegenden) Seiten des zentralen Griffgehäuses 3 angelenkt / anscharniert sind und die sich längs des zentralen Griffgehäuses 3 in Richtung proximal vorzugsweise über das proximale Ende des zentralen Griffgehäuses 3 hinaus erstecken und
- einen elektrischen, vorzugsweise bipolaren Anschluss 15 zum Anschließen einer bzw. zweier nicht weiter dargestellten elektrischen Leitung(en), der/die am proximalen Ende oder Endabschnitt des zentralen Griffgehäuses 3 vorzugsweise zwischen den beiden Griffbranchen 13a, 13b angeordnet ist/sind.

Das zentrale Griffgehäuse 3 hat gemäß der Fig. 1 eine im Wesentlichen langgestreckte, wenngleich vorzugsweise (bananenartig) gekrümmte, zylinderförmige (sowie im Querschnitt ovale) Gestalt, an dessen distalem Endabschnitt die Kupplungs- und Dreheinrichtung 5 beispielsweise gemäß der WO 2011/097469 A2 A1 positioniert ist, deren konstruktiver Aufbau somit allgemein zum Stand der Technik zählt und daher an dieser Stelle nicht weiter beschrieben werden muss. Entscheidend ist, dass die Kupplungs- und Dreheinrichtung 5 dafür vorgesehen und ausgebildet ist, den Instrumentenschaft 7, insbesondere dessen proximales Kupplungsstück 7a aufzunehmen und gegen ein ungewolltes Herausziehen aus dem zentralen Griffgehäuse 3 zu sichern. Außerdem ist innerhalb des Instrumentenschafts 7 in der Regel die Transmission beispielsweise in Form eines Zug-Druckstabs 9 gelagert, über die der distaler Effektor 11 am Instrumentenschaft 7 mechanisch betätigbar ist. Beispielsweise kann ein solcher Effektor 11 zwei scheren- oder zangenförmig zueinander bewegbare Gewebe-Eingriffsbranchen 11a, 11b haben, mittels denen ein Patientengewebe ergriffen werden kann. Außerdem können die Gewebe-Eingriffsbranchen 11a, 11b jeweils mit Elektrodenreihen 11c besetzt sein oder aus einem elektrisch leitenden Material bestehen, um einen elektrischen Strom in das Patientengewebe wahlweise einzuleiten, das zwischen den beiden Branchen 11a, 11b eingeklemmt ist. Auch die Konstruktion eines solchen Effektors 11 ist aus dem Stand der Technik beispielsweise gemäß der WO 2011/097469 A2 A1 hinlänglich bekannt, sodass an dieser Stelle ebenfalls auf diesen Stand der Technik verwiesen werden kann.

Die beweglichen Griffelemente/Griffbranchen 13a, 13b erstrecken sich, wie vorstehend angedeutet, im Wesentlichen in Längsrichtung des zentralen Griffgehäuses 3, also von distal in Richtung proximal und sind nach Art von Scheren- oder Zangengriffbranchen aufeinander zu oder voneinander weg bewegbar/schwenkbar. Die Griffelemente 13a, 13b bilden an ihren proximalen Endabschnitten jeweils sogenannte (ovalförmige) Fingerösen 13c, welche jedoch im vorliegenden Fall nicht geschlossen, sondern in ihren jeweiligen proximalen ÖsenAbschnitten offen sind und somit eine Art U-Form bilden, deren Öffnung nach proximal weist. Am zentralen Griffgehäuse 3 sind an seinen vorstehend genannten, im Wesentlichen diametral sich gegenüberliegenden (Mantel-)Seiten jeweils eine Einstecktasche 3a, 3b ausgeformt, die (nur) in Richtung proximal offen sind und in die die bewegbaren Griffelemente 13a, 13b (quasi von proximal in Richtung distal) eingesteckt sind.

Wie auch in den Fig. 1a, 1b zu erkennen ist, erstrecken sich die beiden beweglichen Griffelemente 13a, 13b nicht exakt koaxial zur (distal-proximalen) Mittellinie des zentralen Griffgehäuses 3, sondern sind in einem Winkel α, vorzugsweise um bis zu 25° - 45° zu dieser (gemäß Fig. 1 nach unten d. h. zusätzlich in Krümmungsrichtung des Griffgehäuses 3) abgekippt, sodass die Fingerösen 13c abschnittsweise unter dem oder zumindest im unteren Bereich des zentralen Griffgehäuses 3 zu liegen kommen.

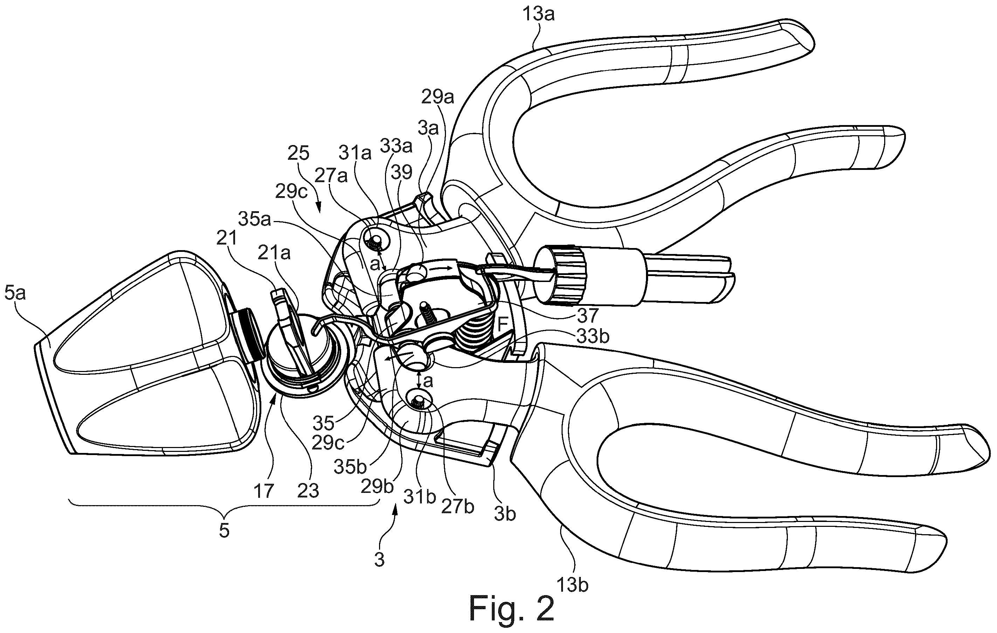

In Fig. 2 ist das zentrale Griffgehäuse 3 im perspektivischen Aufriss dargestellt, wobei die linke Seite der Fig. 2 in Richtung distal und die rechte Seite der Fig. 2 in Richtung proximal weist. Zu sehen ist hier zunächst auf der distalen Seite des Handgriffs/Griffgehäuses 3 eine manuell betätigbare Verrastbaugruppe 17 als Bestandteil der vorstehend genannten Kupplungs- und Dreheinrichtung 5 mit einem im Wesentlichen lotrecht zur in Fig. 1 durch einen Pfeil darstellten Einkupplungsrichtung 19 sich erstreckenden und bewegbaren Schieber 21 und einem daran fixierten Betätigungsknopf 23. Der Schieber 21 ist hier plattenförmig ausgebildet, mit einer mittigen Durchgangsöffnung 21a, in welche der Instrumentenschaft 7 beim Einkuppelvorgang eingesteckt werden kann, sobald der Betätigungsknopf 23 gedrückt (oder alternativ gezogen) ist. Wird der Betätigungsknopf 23 freigegeben, verschiebt sich der Schieber 21 in seine Konstruktionslage zurück (z.B. mittels einer Feder) und verrastet beispielsweise in einer nicht weiter dargestellten Hinterschneidung am Instrumentenschaft 7 insbesondere an dessen Kupplungsstück 7a, sodass dieser nichtmehr aus dem Handgriff 1 gezogen aber immer noch gedreht werden kann. Dabei sei an dieser Stelle darauf hingewiesen, dass die Verrastbaugruppe 17 auch einen anderen konstruktiven Aufbau haben kann, beispielsweise in Form eines Bolzens oder Kugeln, welche in entsprechende Hinterschneidungen oder Kerben am Instrumentenschaft bzw. dessen Kupplungsstück/-abschnitt 7a einrasten können.

Proximal zur Verrastbaugruppe 17 ist ein Getriebe 25 zu sehen, das in dem zentralen Griffgehäuse 3 untergebracht ist und über welches die Schwenkbewegungen der beweglichen Griffelemente 13a, 13b in eine Translationsbewegung des Zug-DruckStabs 9 innerhalb des Instrumentenschafts 7 übertragbar/umwandelbar ist.

Demzufolge sind die beiden beweglichen Griffelemente/Griffbranchen 13a, 13b an Schwenkscharnieren/Schwenkachsen 27a, 27b angelenkt, die innerhalb des zentralen Griffgehäuses 3 im Bereich der Einstecktaschen 3a, 3b angeordnet sind. Die Schwenkachsen 27a, 27b erstrecken sich vorliegend nicht exakt parallel, sondern in einem spitzen Winkel zueinander sowie voneinander weg (also V-förmig) und spannen gemeinsam eine Ebene auf, welche die distal-proximal-Mittellinie M des zentralen Griffgehäuses 3 im Wesentlichen rechtwinklig schneidet. Dabei ist mit dem Begriff "im Wesentlichen" auch noch ein Abkippen dieser Ebene bezüglich der distal-proximal-Mittellinie M in Richtung proximal um einen Winkel von bis zu 25° - 40° mit umfasst.

Der Einfachheit halber wird nachstehend davon gesprochen, dass die Einkupplungsrichtung 19 ungefähr der distal-proximal-Richtung (vorn/hinten) angenähert ist, die Richtung, in welcher die Schwenkachsen beabstandet sind, ungefähr der Breitenrichtung (links/rechts) des Instruments / Handgriffs 1 angenähert ist und die Richtung, in welche sich die Schwenkachsen 27a, b jeweils erstrecken, ungefähr der Tiefenrichtung (oben/unten) angenähert ist.

Die Griffbranchen 13a, b bilden im Bereich der Schwenkachsen 27a, b sogenannte Scharnierköpfe 29a, 29b aus, in die Lagerungsbohrungen (oben-unten) 31a, 31b zur Drehaufnahme der Schwenkachsen 29a, b ausgeformt sind. Senkrecht zu diesen Lagerungsbohrungen 31a, b sind in den Scharnierköpfen 29a, b (in Breitenrichtung sich erstreckende) Aufnahme- bzw. Kugelkopftaschen 33a, 33b ausgebildet, die im montierten Zustand beider Griffbranchen 13a, b einander zugewandt sind. In dem Griffgehäusebereich zwischen den beiden Scharnierköpfen 29a, b ist ein (in Breitenrichtung sich erstreckender) Querstab 35 platziert, an dessen Enden jeweils eine Kugel 35a, 35b angeformt ist, die in den Kugelkopftaschen 33a, b (in Breitenrichtung) gleitend und (um die Querstabachse) drehend aufgenommen sind. Aufgrund des, einen Hebelarm bildenden Abstands a zwischen der Kugelkopftasche bzw. der darin gelagerten Kugel 35a, b und der Lagerungsbohrung 27a, b jeder Griffbranche 13a, b bewirkt eine Schwenkbewegung der Fingeröse 13c jeder Griffbranche 13a, b hin zu und weg vom zentralen Griffgehäuse 3 eine Translationsbewegung der Querstange 35 im Wesentlichen entlang der Einkupplungsrichtung 19.

Zwischen den beiden Kugeln 35a, b ist ein Hebel 37 am Querstab 35 drehbar um diesen angelenkt, der sich bei Konstruktionslage der Griffbranchen 13a, b gemäß Fig. 2 im Wesentlichen lotrecht oder in einem (stumpfen) Winkel zur Einkupplungsrichtung 19 in Tiefenrichtung (nach oben) erstreckt und in seinem Mittenabschnitt am zentralen Griffgehäuse 3 wippenartig gelagert ist. Wird also der Querstab 35 und damit der mit dem Querstab 35 schwenkgekoppelte (Querstabzugewandte) Wippenteil des Hebels 37 beispielsweise bei Zusammenrücken der Griffbranchen 13a, 13b (Fingerösen 13c) gemäß der Fig. 2 in Richtung distal (nach vorn) bewegt (geschwenkt), bewegt sich (schwenkt) der freie Endabschnitt des anderen (Querstab-abgewandten) Wippenteils des Hebels 37 folglich in Richtung proximal (nach hinten) und drückt dabei eine Feder F zusammen. Diese Bewegungsrichtungen sind in Fig. 2 durch zwei gegenläufige Pfeile dargestellt.

Der Hebel 37 weist an seinem dem Querstab 35 abgewandten Hebelende (also am freien Endabschnitt des anderen Wippenteils des Hebels 37) einen durchgehenden Aufnahme-Lagerungsschlitz 39 auf, der sich in Tiefenrichtung (oben-unten) erstreckt und in den ein Lagerkopf oder Lagerbozen 9a am proximalen Ende des Zug-Druckstabs 9 (siehe Fig. 1) lösbar eingesetzt/eingeschoben ist bzw. einsetzbar/einschiebbar ist. Zu diesem Zweck können die beiden beweglichen Griffelemente 13a, b über die in der Fig. 2 dargestellte Konstruktionslage (Betriebsendposition) hinaus zusätzlich voneinander weg in eine Einkuppelschwenkposition verschwenkt werden, wodurch sich der Querstab 35 und damit der freie sowie geschlitzte Endabschnitt des Hebels 37 über die in Fig. 2 gezeigte Konstruktionslage hinausbewegen. D.h., der freie Endabschnitt des Hebels 35 mit Schlitz 39 wird weiter in Richtung distal verschwenkt, als dies die Konstruktions-/Betriebslagelage/Betriebsendposition gemäß der Fig. 2 vorsieht, wobei sich natürlich/zwangsläufig der zumindest oben offene Schlitz 39 in Richtung distal neigt, wodurch die obere Schlitzöffnung aus der Richtung distal zugänglich wird. In dieser Einkuppelschwenkposition (jenseits der Betriebsendposition) lässt sich der proximale Lagerbolzen 9a am Zug-Druckstab 9 von distal in Richtung proximal in den Schlitz 39 einschieben und damit an den Hebel 35 koppeln und natürlich auch wieder entkoppeln. Sobald die beiden Griffbranchen 13a, b wieder aus der Einkuppelschwenkposition in die Konstruktions-/Betriebslage / Betriebsendposition gemäß der Fig. 2 zurückgeschwenkt sind, kann der Lagerbolzen 9a am Zug-Druckstab 9 nichtmehr aus dem Schlitz 39 im Hebel 37 gezogen werden.

Um zu verhindern, dass die Griffbranchen 13a, b unbeabsichtigt die Einkuppelschwenkposition annehmen, kann am Handgriff 1 eine nicht weiter gezeigte, manuell betätigbare Sperre, beispielsweise ein (eindrückbarer oder abziehbarer) Anschlagbolzen oder dergleichen vorgesehen sein. Alternativ sind aber auch andere Mechanismen denkbar, etwa der Instrumentenschaft 7 selbst, der sich gegen Ende des Einkuppelvorgangs zwischen die Griffbranchen 13a, b (längs-)schiebt und somit ein über die Konstruktionslage hinausgehendes Aufschwenken der Griffbranchen 13a, b verhindert. Zu diesem Zweck können an den Scharnierköpfen 29a, b der beiden Griffbranchen 13a, b (oder nur einer Griffbranche) nach innen ragende Vorsprünge 29c ausgebildet sein (also distal zu den Dreh-/Schwenkachsen), die sich bei Aufschwenken der Griffbranchen 13a, b nach innen bewegen und sich schließlich am Instrumentenschaft 7 abstützen, um so die Aufschwenkbewegung (in Richtung hin zur Einkuppelschwenkposition) zu begrenzen/stoppen. Im Umkehrzug wird beim Entkoppeln des Instrumentenschafts 7 vom Handgriff 1 dieser zuerst (zu Beginn des Entkopplungsvorgangs) aus dem Handgriff 1 gezogen, zumindest soweit, bis die nach innen ragenden Vorsprünge 29c an den Scharnierköpfen 29a, b der Griffbranchen 13a, b nichtmehr am Instrumentenschaft 7 anliegen können (also an diesem nach innen vorbeischwenken können) und damit ein weiteres Aufschwenken der Griffbranchen 13a, b über die Betriebsendposition gemäß Fig. 2 hinaus möglich wird. In dieser Schwenkposition neigt sich der Einkoppel-Schlitz am freien Ende des Hebels in Richtung proximal, wodurch die (obere) Schlitzöffnung in Richtung distal ausgerichtet wird. Letztlich ist es aber auch möglich, die Mechanik des Effektors 11 als Anschlag zu benutzen, welche nur dann funktioniert, wenn der Instrumentenschaft 7 eingekuppelt ist.

Die wesentlichen sowie separat beanspruchbaren Aspekte der Offenbarung gemäß dem in Fig. 2 dargestellten Ausführungsbeispiels sind u.a. also
- die beidseitige Anordnung der Betätigungsbranchen 13a, b bezüglich des zentralen Griffgehäuses 3,
- die V-förmige Anstellung der Schwenkachsen 27a, b zur Simulation/Annäherung der kreisförmigen Fingerbewegung einer sich schließenden menschlichen Hand,
- die gleitende sowie drehende Lagerung des Querstabs 35 in den Aufnahmetaschen33a, b der Betätigungsbranchen 13a, b sowie die Lagerung des Wippenhebels 37 am Querstab 35, um dadurch eine Schließbewegung der Betätigungsbranchen aufeinander zu in eine Zugbewegung des Zug-Druck-Stabs 9 innerhalb des Instrumentenschafts 7 zu transformieren und
- die direkte oder indirekte Anschlagfunktion des eingekuppelten Instrumentenschafts oder dessen Effektors zur Begrenzung der Schwenkbewegung der Betätigungsbranchen vor Erreichen einer Position, in welcher der Zug-Druckstab 9 vom Getriebe, insbesondere vom Wippenhebel 37 innerhalb des zentralen Griffgehäuses ein- oder abkoppelbar ist.

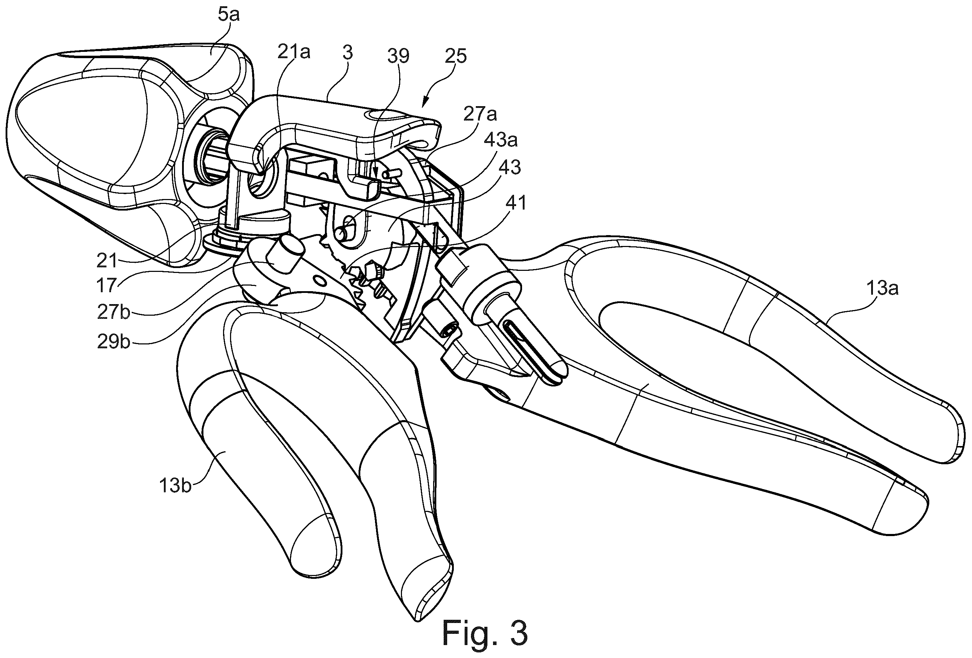

In der Fig. 3 ist ein zum Ausführungsbeispiel gemäß der Fig. 2 alternatives Getriebe 25 eines offenbarungsgemäßen Handgriffs 1 gezeigt, wobei für zum ersten Ausführungsbeispiel gemäß Fig. 2 gleiche Bauteile auch weiterhin die gleichen Bezugszeichen verwendet werden.

Im distalen Abschnitt des zentralen Griffgehäuses 3 ist wiederum die Verrastbaugruppe 17 zu erkennen, an die sich proximal dazu das im zentralen Griffgehäuse verbaute Getriebe 25 anschließt. Beidseits gegenüberliegend sind die beweglichen Griffelemente 13a, b dargestellt mit den distal angeordneten Scharnierköpfen 29a, 29b, die auf die entsprechenden stiftartigen Scharnierachsen 27a, b aufgesteckt sind. An den einander zugewandten (Umfangs-) Randbereichen der Scharnierköpfe 29a, b (nur einer ist gezeigt) sind Teilkreis-Zahnräder 41 montiert (nur ein Teilkreis-Zahnrad 41 ist in der Fig. 3 dargestellt, wohingegen das gegenüberliegende Teilkreis-Zahnrad verdeckt ist), deren Zähne jeweils einander zugewandt sind, sich also nach innen erstrecken und deren Drehachsen durch die Schwenkachsen 27a, 27b gebildet sind.

Zwischen den Teilkreis-Zahnrädern 41 ist ein Zentralzahnrad 43 am zentralen Griffgehäuse 3 gelagert, dessen Drehachse 43a im Wesentlichen senkrecht zu den Schwenkachsen 27a, b ausgerichtet ist, sich also in Querrichtung / Breitenrichtung des Handgriffs 1 gemäß vorstehender Definition erstreckt und mit welchem die Teilkreis-Zahnräder 41 in Wirkeingriff stehen. Zu erwähnen ist an dieser Stelle noch, dass sich die Drehachse 43a des Zentralzahnrads 43 oberhalb (gemäß vorstehender Definition) der Teilkreis-Zahnräder 41 befindet und der Aufnahme-Lagerungsschlitz 39 oberhalb der Drehachse 43a in das Zentralzahnrad 43 eingearbeitet ist. In Übereinstimmung zur Funktionsweise des ersten Ausführungsbeispiels wird dadurch erreicht, dass bei einer Schwenkbewegung der Griffbranchen 13a, b beispielsweise aufeinander zu (Zusammendrücken der beiden Griffbranchen 13a, b) das Zentralzahnrad 41 als Bewegungsrichtungsumkehrmittel vergleichbar zu dem Hebel 37 des ersten Ausführungsbeispiels genutzt wird, um diese Griffbranchenbewegung in eine Zugbewegung an der Zug-Druckstange 9 innerhalb des Instrumentenschafts 7 zu transformieren (also in Richtung proximal).

Grundsätzlich ist es bei beiden Ausführungsbeispielen gemäß der Offenbarung vorgesehen, dass die durch den Hebel 37 bzw. durch das Zentralzahnrad 43 erzeugten Hebelarme zwischen Schwenkkopf 29a, b und dem Aufnahme-Lagerungsschlitz 39 ein Längenverhältnis von 1:1 aufweisen, wodurch die Schwenkbewegung der Griffbranchen 13a, b ohne Übersetzung in die Translationsbewegung des Zug-Druckstabs 9 übertragen wird. Es ist aber auch möglich, durch entsprechende Gestaltung des Hebels 37 (Wippenteillängen) gemäß dem ersten Ausführungsbeispiel oder des Zentralzahnrads 43 (Zahnraddurchmesser) des zweiten Ausführungsbeispiels eine Über- oder Untersetzung der Schwenkbewegung der Griffbranchen 13a, b zu erreichen.

Zusammengefasst ist ein Handgriff eines oder für ein chirurgisches Instrument, insbesondere elektrochirurgischen Instrument der minimalinvasiven Schaftbauart offenbart mit
- einem zentralen Griffgehäuse 3, das sich von distal in Richtung proximal erstreckt,
- einer kombinierten Kupplungs- und Dreheinrichtung 5 am distalen Endabschnitt des Griffgehäuses 3, die zur wahlweisen Ankupplung sowohl eines Instrumentenschafts 7 an das zentrale Griffgehäuse 3 als auch einer im Instrumentenschaft 7 gelagerten Transmission 9 an ein im zentralen Griffgehäuse 3 aufgenommenes Getriebe 25 vorgesehen und ausgebildet ist,
- zwei diametral gegenüberliegend am Griffgehäuse 3 positionierten Griffbranchen 13a, 13b, die sich beidseits des zentralen Griffgehäuses 3 von distal in Richtung proximal erstrecken und die an ihren distalen Endabschnitten am Griffgehäuse 3 angelenkt sind, derart, dass sie aufeinander zu und voneinander weg bewegbar sind und
- Kraftübertragungselementen oder Kraftübertragungsabschnitten 33a, 33b, 41 (vorliegend vorzugsweise die Aufnahmetaschen oder die Teilkreiszahnräder), die am distalen Endabschnitt jeder Griffbranche 13a, 13b ausgebildet oder angeordnet sind und mit dem Getriebe 25 in Wirkeingriff stehen, um die Griffbranchenbewegung auf die Transmission 9 (also Translationsbewegung) zu übertragen.

Ferner ist ein chirurgisches Instrument, insbesondere elektrochirurgisches Instrument der minimalinvasiven Schaftbauart offenbart mit einem Instrumentenschaft 7, in dem eine Transmission 9 vorzugsweise in Form eines Zug-Druckstabs gelagert ist und an dessen distalem Endabschnitt ein Effektor 11 angeordnet ist, der über die Transmission 9 mechanisch betätigbar und ggf. über elektrische Leiter mit einem elektrischen Strom beaufschlagbar ist, wobei der Instrumentenschaft 7 und die darin gelagerte Transmission 9 mit dem vorstehend genannten Handgriff 3 als Bestandteil des chirurgischen Instruments gekoppelt oder koppelbar sind (d.h. der Instrumentenschaft 7 mit dem zentralen Griffgehäuse 3 und die Transmission 9 mit dem im Griffgehäuse 3 untergebrachten Getriebe 25 gekoppelt oder koppelbar sind).

## Patentansprüche

1. Handgriff eines oder für ein chirurgisches Instrument, insbesondere elektrochirurgisches Instrument der minimalinvasiven Schaftbauart, mit
- einem zentralen Griffgehäuse (3), das sich von distal in Richtung proximal erstreckt,
- einem im zentralen Griffgehäuse (3) aufgenommenen Getriebe (25),
- einer kombinierten Kupplungs- und Dreheinrichtung (5) am distalen Endabschnitt des zentralen Griffgehäuses (3), die zur wahlweisen Ankupplung sowohl eines Instrumentenschafts (7) an das zentrale Griffgehäuse (3) als auch einer im Instrumentenschaft (7) gelagerten Transmission (9) an das Getriebe (25) vorgesehen und ausgebildet ist,
- zwei diametral gegenüberliegend am Griffgehäuse (3) positionierte Griffbranchen (13a, 13b), die sich beidseits des zentralen Griffgehäuses (3) von distal in Richtung proximal erstrecken und die an ihren distalen Endabschnitten am zentralen Griffgehäuse (3) angelenkt sind, derart, dass sie aufeinander zu und voneinander weg bewegbar sind,
- Kraftübertragungselemente oder Kraftübertragungsabschnitte (33a, 33b, 41), die am distalen Endabschnitt jeder Griffbranche (13a, 13b) ausgebildet oder angeordnet sind und mit dem Getriebe (25) in Wirkeingriff stehen, um die Griffbranchenbewegung auf die Transmission (9) zu übertragen, und
- einem Querstab (35), der in Wirkeingriff mit den Kraftübertragungsabschnitten (33a, 33b) der beiden Griffbranchen (13a, 13b) steht.

2. Handgriff nach Anspruch 1, **gekennzeichnet durch** zwei diametral gegenüberliegend am/im zentralen Griffgehäuse (3) angeordnete Schwenkachsen (27a, 27b), an denen die Griffbranchen (13a, 13b) angelenkt sind.

3. Handgriff nach Anspruch 2, **dadurch gekennzeichnet, dass** die Kraftübertragungsabschnitte (33a, 33b) in Form von Aufnahmetaschen ausgebildet sind, welche in den distalen Endabschnitten der beiden Griffbranchen (13a, 13b) jeweils im Abstand (a) zu den Schwenkachsen (27a, 27b) ausgeformt sowie einander zugewandt sind.

4. Handgriff nach Anspruch 3, **dadurch gekennzeichnet, dass** der Querstab (35) an beiden Endabschnitten mit Kugeln oder Teilkugeln (35a, 35b) ausgebildet oder versehen ist, die in den Kraftübertragungsabschnitten (33a, 33b), insbesondere in den beiden Aufnahmetaschen (33a, 33b) drehend sowie querstab-axial gleitend gelagert sind.

5. Handgriff nach einem der Ansprüche 1 bis 4, **dadurch gekennzeichnet, dass** am Querstab (35) ein wippenartig am/im zentralen Griffgehäuse (3) gelagerter Hebel (37) angelenkt ist, an dem, insbesondere an dessen dem Querstab (35) abgewandten freien Endabschnitt, eine Kupplungsaufnahme vorzugsweise in Form eines Schlitzes (39) für die Transmission (9) ausgebildet oder angeordnet ist,
wobei vorzugsweise ein an dem Querstab (35) angelenktes Ende des Hebels (37) und/oder eine Querstabachse auf einer Kreisbahn um eine Wippenachse des Hebels (37) beweglich sind, wobei eine Wippenachse des Hebels (37) vorzugsweise senkrecht zu einer distal-proximal-Mittellinie und/oder zu einer Tiefenrichtung und/oder parallel zu einer Breitenrichtung ist, wobei die Tiefenrichtung und die Breitenrichtung des Handgriffs senkrecht zueinander sowie im Wesentlichen senkrecht zu der distal-proximal-Mittellinie des Handgriffs sind.

6. Handgriff nach einem der Ansprüche 2 bis 5, **dadurch gekennzeichnet, dass** die Schwenkachsen (27a, 27b) in einem spitzen Winkel vorzugsweise zwischen 0° und 25° zueinander angestellt sind und zwar in einer Anstellebene, welche eine distal-proximal-Mittellinie des zentralen Griffgehäuses (3) in einem Winkel kleiner-gleich 90°, vorzugsweise zwischen 90° und 65° schneidet.

7. Handgriff nach einem der Ansprüche 2 bis 6, **dadurch gekennzeichnet, dass** eine Tiefenrichtung und eine Breitenrichtung des Handgriffs senkrecht zueinander sowie im Wesentlichen senkrecht zu einer distal-proximal-Mittellinie des Handgriffs sind, wobei die Schwenkachsen jeweils, betrachtet in einer Ebene enthaltend die Tiefenrichtung und die Breitenrichtung, zur Tiefenrichtung im spitzen Winkel, insbesondere von 0° bis 25°, vorzugsweise von 5 bis 15°, geneigt sind und/oder jeweils, betrachtet in einer Ebene enthaltend die Tiefenrichtung und die distal-proximal-Mittellinie, zur Tiefenrichtung im spitzen Winkel, insbesondere von 0 bis 25°, vorzugsweise von 5 bis 20°, geneigt sind.

8. Handgriff nach einem der Ansprüche 3 bis 7, **dadurch gekennzeichnet, dass** die distalen Endabschnitte jeder Griffbranche (13a, 13b) mit nach innen aufeinander zu ausgerichteten Vorsprüngen (29c) ausgebildet oder versehen sind, welche dafür vorgesehen und ausgebildet sind, als Aufschwenkbegrenzungen für die beiden Griffbranchen (13a, 13b) zu dienen, indem diese gegen den bereits angekuppelten Instrumentenschaft (7) innerhalb des zentralen Griffgehäuses (3) anschlagen,
wobei vorzugsweise bei Abkuppeln des Instrumentenschafts (7) vom Handgriff (3) die Aufschwenkbegrenzung entfällt und damit ein zusätzliches Aufschwenken der beiden Griffbranchen (13a, 13b) in eine Einkupplungsschwenkposition erlaubt ist, in der die Transmission (9) mit der Kupplungsaufnahme vorzugsweise in Form eines Schlitzes (39) in und außer Eingriff bringbar ist.

9. Handgriff nach einem der Ansprüche 3 bis 8, **dadurch gekennzeichnet, dass** die Kraftübertragungsabschnitte (33a,33b) in Tiefenrichtung zumindest einseitig offen, vorzugsweise zu einer Handgriffoberseite hin, offen ausgebildet sind.

10. Handgriff nach einem der Ansprüche 1 bis 9, **dadurch gekennzeichnet, dass** die Griffbranchen (13a, 13b) jeweils eine Fingeröse (13c) aufweisen, die an ihren jeweiligen proximalen Ösenabschnitten umfangsseitig offen ausgebildet sind, insbesondere im Wesentlichen eine nach proximal geöffnete U-Form aufweisen.

11. Handgriff nach einem der Ansprüche 1 bis 10, **dadurch gekennzeichnet, dass** die Kupplungs- und Dreheinrichtung (5) einen Schaft-Kupplungsabschnitt zur wahlweisen Ankupplung, insbesondere axial/translationsfesten und drehbaren Aufnahme, des Instrumentenschafts (7) an dem Griffgehäuse (3) und einen Transmissions-Kupplungsabschnitt zur wahlweisen Ankupplung, insbesondere axial/translationsfesten Aufnahme, der Transmission (9) an das Getriebe (25) aufweisen.

12. Handgriff nach einem der Ansprüche 1 bis 11, **dadurch gekennzeichnet, dass** die Griffbranchen (13a, 13b) jeweils zu einer anderen Griffbranche der zwei Griffbranchen (13a, 13b) schwenkbar an dem Griffgehäuse (3) angelenkt sind und das Getriebe (25) einen mit den Griffbranchen (13a, 13b) gekoppelten oder koppelbaren Griffbranchen-Kupplungsabschnitt aufweist und ausgebildet ist, um, insbesondere im an den Handgriff gekoppelten Zustand des Instrumentenschafts (7) und der Transmission (9), eine Schwenkbewegung der Griffbranchen (13a, 13b) in eine Translationsbewegung der Transmission (9) zu übertragen.

13. Handgriff eines oder für ein chirurgisches Instrument, insbesondere elektrochirurgisches Instrument der minimalinvasiven Schaftbauart, mit
- einem zentralen Griffgehäuse (3), das sich von distal in Richtung proximal erstreckt,
- einem im zentralen Griffgehäuse (3) aufgenommenen Getriebe (25),
- einer kombinierten Kupplungs- und Dreheinrichtung (5) am distalen Endabschnitt des zentralen Griffgehäuses (3), die zur wahlweisen Ankupplung sowohl eines Instrumentenschafts (7) an das zentrale Griffgehäuse (3) als auch einer im Instrumentenschaft (7) gelagerten Transmission (9) an das Getriebe (25) vorgesehen und ausgebildet ist,
- zwei diametral gegenüberliegend am Griffgehäuse (3) positionierte Griffbranchen (13a, 13b), die sich beidseits des zentralen Griffgehäuses (3) von distal in Richtung proximal erstrecken und die an ihren distalen Endabschnitten am zentralen Griffgehäuse (3) angelenkt sind, derart, dass sie aufeinander zu und voneinander weg bewegbar sind,
- Kraftübertragungselemente oder Kraftübertragungsabschnitte (33a, 33b, 41), die am distalen Endabschnitt jeder Griffbranche (13a, 13b) ausgebildet oder angeordnet sind und mit dem Getriebe (25) in Wirkeingriff stehen, um die Griffbranchenbewegung auf die Transmission (9) zu übertragen,
- zwei diametral gegenüberliegend am/im zentralen Griffgehäuse (3) angeordnete Schwenkachsen (27a, 27b), an denen die Griffbranchen (13a, 13b) angelenkt sind,
wobei die Kraftübertragungsabschnitte (41) in Form von Zahnrädern oder Teilkreiszahnrädern ausgebildet sind, die mit einem Zentralzahnrad (43) in Kämmeingriff stehen, dessen Drehachse (43a) parallel zu einer die Schwenkachsen (27a, 27b) verbindenden gedachten Linie ausgerichtet ist und welches eine Kupplungsaufnahme vorzugsweise in Form eines Schlitzes (39) für die Transmission (9) aufweist.

14. Chirurgisches Instrument, insbesondere elektrochirurgisches Instrument der minimalinvasiven Schaftbauart mit einem Instrumentenschaft (7), in dem eine Transmission (9) vorzugsweise in Form eines Zug-Druckstabs gelagert ist und an dessen distalem Endabschnitt ein Effektor (11) angeordnet ist, der über die Transmission (9) mechanisch betätigbar und ggf. über elektrische Leiter mit einem elektrischen Strom beaufschlagbar ist, **gekennzeichnet durch** einen Handgriff (3) nach einem der Ansprüche 1 bis 13, wobei der Instrumentenschaft (7) und die darin gelagerte Transmission (9) mit dem Handgriff (3) gekoppelt oder koppelbar ist, insbesondere der Instrumentenschaft (7) mit dem zentralen Griffgehäuse (3) und die Transmission (9) mit dem im zentralen Griffgehäuse (3) untergebrachten Getriebe (25) gekoppelt oder koppelbar sind.

## Claims

1. A handle of or for a surgical instrument, in particular an electrosurgical instrument of the minimally invasive shaft design, with
- a central grip housing (3) extending from the distal in the proximal direction,
- a gear unit (25) received in the central grip housing (3),
- a combined coupling and rotating device (5) at the distal end portion of the central grip housing (3), which is provided and configured for selectively coupling both an instrument shaft (7) to the central grip housing (3) and a transmission (9) mounted in the instrument shaft (7) to the gear unit (25),
- two grip branches (13a, 13b) positioned diametrically opposite each other on the grip housing (3), which extend on both sides of the central grip housing (3) from the distal in the proximal direction and which are hinged to the central grip housing (3) at their distal end portions in such a way that they can be moved toward and away from each other,
- force-transmitting elements or force-transmitting portions (33a, 33b, 41) which are configured or arranged at the distal end portion of each grip branch (13a, 13b) and which are in operative engagement with the gear unit (25) in order to transmit the grip-branch movement to the transmission (9), und
- a crossbar (35) which is in operative engagement with the force-transmitting portions (33a, 33b) of the two grip branches (13a, 13b).

2. The handle according to claim 1, **characterized by** two pivot axes (27a, 27b) arranged diametrically opposite each other on/in the central grip housing (3), on which the grip branches (13a, 13b) are hinged.

3. The handle according to claim 2, **characterized in that** the force-transmitting portions (33a, 33b) are configured in the form of receiving pockets which are formed in the distal end portions of the two grip branches (13a, 13b) respectively at a distance (a) from the pivot axes (27a, 27b) and facing each other.

4. The handle according to claim 3, **characterized in that** the crossbar (35) is configured or provided with balls or partial balls (35a, 35b) at both end portions, which are mounted in the force-transmitting portions (33a, 33b), in particular in the two receiving pockets (33a, 33b), in a rotating and crossbar-axially sliding manner.

5. The handle according to one of the claims 1 to 4, **characterized in that** a lever (37) mounted in a rocker-like manner on/in the central grip housing (3) is hinged to the crossbar (35), wherein a coupling receptacle for the transmission (9) is configured or arranged on the lever (37), in particular on its free end portion facing away from the crossbar (35), preferably in the form of a slit (39),
wherein, preferably an end of the lever (37) hinged to the crossbar (35) and/or a crossbar axis are movable on a circular path around a rocker axis of the lever (37), wherein a rocker axis of the lever (37) is preferably perpendicular to a distal-proximal center line and/or to a depth direction and/or parallel to a width direction, wherein the depth direction and the width direction of the handle are perpendicular to each other and substantially perpendicular to the distal-proximal center line of the handle.

6. The handle according to one of claims 2 to 5, **characterized in that** the pivot axes (27a, 27b) are angled at an acute angle, preferably between 0° and 25°, to each other in an angled plane which intersects a distal-proximal center line of the central grip housing (3) at an angle less than or equal to 90°, preferably between 90° and 65°.

7. The handle according to one of claims 2 to 6, **characterized in that** a depth direction and a width direction of the handle are perpendicular to each other and substantially perpendicular to a distal-proximal center line of the handle, wherein the pivot axes are each inclined at an acute angle to the depth direction, in particular from 0° to 25°, preferably from 5 to 15°, when viewed in a plane containing the depth direction and the width direction, and/or are each inclined at an acute angle to the depth direction, in particular from 0 to 25°, preferably from 5 to 20°, when viewed in a plane containing the depth direction and the distal-proximal center line.

8. The handle according to one of claims 3 to 7, **characterized in that** the distal end portions of each grip branch (13a, 13b) are configured or provided with projections (29c) aligned inward toward each other, which are provided and configured to serve as pivot limitations for the two grip branches (13a, 13b) by abutting against the already coupled instrument shaft (7) within the central grip housing (3),
Wherein, preferably when the instrument shaft (7) is uncoupled from the handle (3), the pivot limitation is omitted and thus an additional pivoting of the two grip branches (13a, 13b) into a coupling pivot position is permitted, in which the transmission (9) can be brought into and out of engagement with the coupling receptacle, preferably in the form of a slit (39).

9. The handle according to one of claims 3 to 8, **characterized in that** the force-transmitting portions (33a, 33b) are configured to be open in the depth direction at least on one side, preferably toward an upper handle side.

10. The handle according to one of claims 1 to 9, **characterized in that** the grip branches (13a, 13b) each have a finger loop (13c), which are configured to be open on the circumferential side at their respective proximal loop portions, in particular substantially having a proximally open U-shape.

11. The handle according to one of claims 1 to 10, **characterized in that** the coupling and rotating device (5) comprises a shaft coupling portion for selectively coupling, in particular axially/translationally fixed and rotatably receiving, of the instrument shaft (7) to the grip housing (3) and a transmission coupling portion for selectively coupling, in particular axially/translationally fixed receiving, of the transmission (9) to the gear unit (25).

12. The handle according to one of claims 1 to 11, **characterized in that** the grip branches (13a, 13b) are each pivotably hinged to a different grip branch of the two grip branches (13a, 13b) to the grip housing (3), and the gear unit (25) comprises a grip branch-coupling portion coupled or couplable to the grip branches (13a, 13b) and is configured to translate, in particular in the state of the instrument shaft (7) and the transmission (9) coupled to the handle, a pivot movement of the grip branches (13a, 13b) into a translational movement of the transmission (9).

13. A handle of or for a surgical instrument, in particular an electrosurgical instrument of the minimally invasive shaft design, with
- a central grip housing (3) extending from the distal in the proximal direction,
- a gear unit (25) received in the central grip housing (3),
- a combined coupling and rotating device (5) at the distal end portion of the central grip housing (3), which is provided and configured for selectively coupling both an instrument shaft (7) to the central grip housing (3) and a transmission (9) mounted in the instrument shaft (7) to the gear unit (25),
- two grip branches (13a, 13b) positioned diametrically opposite each other on the grip housing (3), which extend on both sides of the central grip housing (3) from the distal in the proximal direction and which are hinged to the central grip housing (3) at their distal end portions in such a way that they can be moved toward and away from each other,
- force-transmitting elements or force-transmitting portions (33a, 33b, 41) which are configured or arranged at the distal end portion of each grip branch (13a, 13b) and which are in operative engagement with the gear unit (25) in order to transmit the grip-branch movement to the transmission (9),
- two pivot axes (27a, 27b) arranged diametrically opposite each other on/in the central grip housing (3), on which the grip branches (13a, 13b) are hinged,
wherein the force-transmitting portions (41) are configured in the form of gearwheels or pitch circle gearwheels which are in meshing engagement with a central gearwheel (43) whose rotation axis (43a) is aligned parallel to an imaginary line connecting the pivot axes (27a, 27b) and which has a coupling receptacle, preferably in the form of a slit (39), for the transmission (9).

14. A surgical instrument, in particular electrosurgical instrument of the minimally invasive shaft design with an instrument shaft (7), in which a transmission (9) is mounted, preferably in the form of a pull-push rod, and at the distal end portion of which an effector (11) is arranged, which is mechanically actuatable via the transmission (9) and, if applicable, an electric current can be applied to it via electrical conductors, **characterized by** a handle (3) according to one of claims 1 to 13, wherein the instrument shaft (7) and the transmission (9) mounted therein are coupled or couplable to the handle (3), in particular the instrument shaft (7) is coupled or couplable to the central grip housing (3) and the transmission (9) is coupled or couplable to the gear unit (25) housed in the central grip housing (3).

## Revendications

1. Poignée d'un ou pour un instrument chirurgical, en particulier un instrument électrochirurgical à tige mini-invasive, avec
- un boîtier de préhension (3) central qui s'étend d'un point distal à proximal ;
- un engrenage (25) logé dans le boîtier de préhension (3) central,
- un dispositif de couplage et de rotation (5) combiné au niveau de la section d'extrémité distale du boîtier de préhension (3) central qui est prévu et configuré pour le couplage au choix aussi bien d'une tige d'instrument (7) au niveau du boîtier de préhension (3) central que d'une transmission (9) logée dans la tige d'instrument (7) au niveau de l'engrenage (25),
- deux branches de préhension (13a, 13b) positionnées diamétralement à l'opposé au niveau du boîtier de préhension (3) qui s'étendent de part et d'autre du boîtier de préhension (3) central d'une position distale dans la direction proximale et qui sont articulées au niveau de leurs sections d'extrémité distales au niveau du boîtier de préhension (3) central de telle manière qu'elles soient mobiles l'une vers l'autre et loin l'une de l'autre,
- des éléments de transmission de force ou des sections de transmission de force (33a, 33b, 41) qui sont configurées ou agencées au niveau de la section d'extrémité distale de chaque branche de préhension (13a, 13b) et sont en prise active avec l'engrenage (25) afin de transmettre le mouvement de branche de préhension à la transmission (9) et
- une barre transversale (35) qui est en prise active avec les sections de transmission de force (33a, 33b) des deux branches de préhension (13a, 13b).

2. Poignée selon la revendication 1, **caractérisée par** deux axes de pivotement (27a, 27b) agencés de manière diamétralement opposée au niveau/dans le boîtier de préhension (3) central, auxquels les branches de préhension (13a, 13b) sont articulées.

3. Poignée selon la revendication 2, **caractérisée en ce que** les sections de transmission de force (33a, 33b) sont configurées sous la forme de poches de réception qui sont déformées dans les sections d'extrémité distales des deux branches de préhension (13a, 13b) respectivement à distance (a) par rapport aux axes de pivotement (27a, 27b) et sont tournées l'une vers l'autre.

4. Poignée selon la revendication 3, **caractérisée en ce que** la barre transversale (35) est configurée ou pourvue au niveau des deux sections d'extrémité de sphères ou sphères partielles (35a, 35b) qui sont logées dans les sections de transmission de force (33a, 33b), en particulier dans les deux poches de réception (33a, 33b) en rotation ainsi qu'en glissement axialement par rapport à la barre transversale.

5. Poignée selon l'une quelconque des revendications 1 à 4, **caractérisée en ce qu'**un levier (37) logé à la manière d'une bascule au niveau/dans le boîtier de préhension (3) central est articulé à la barre transversale (35), levier au niveau duquel, en particulier au niveau de sa section d'extrémité libre éloignée de la barre transversale (35), un logement de couplage est configuré ou agencé de préférence sous la forme d'une fente (39) pour la transmission (9),
dans lequel une extrémité du levier (37) articulée à la barre transversale (35) et/ou un axe de barre transversale sont de préférence mobiles sur une trajectoire circulaire autour d'un axe de bascule du levier (37), dans lequel un axe de bascule du levier (37) est de préférence perpendiculaire à une ligne médiane distale-proximale et/ou à un sens de profondeur et/ou parallèle à un sens de largeur, dans lequel le sens de profondeur et le sens de largeur de la poignée sont perpendiculaires l'un à l'autre et sensiblement perpendiculaires à la ligne médiane distale-proximale de la poignée.

6. Poignée selon l'une quelconque des revendications 2 à 5, **caractérisée en ce que** les axes de pivotement (27a, 27b) sont inclinés selon un angle aigu de préférence entre 0° et 25° l'un à l'autre et ce dans un plan d'inclinaison qui coupe une ligne médiane distale-proximale du boîtier de préhension (3) central selon un angle inférieur ou égal à 90°, de préférence entre 90° et 65°.

7. Poignée selon l'une quelconque des revendications 2 à 6, **caractérisée en ce qu'**un sens de profondeur et un sens de largeur de la poignée sont perpendiculaires l'un à l'autre et sensiblement perpendiculaires à une ligne médiane distale-proximale de la poignée, dans laquelle les axes de pivotement sont inclinés respectivement, considéré dans un plan contenant le sens de profondeur et le sens de largeur, par rapport au sens de profondeur selon l'angle aigu, en particulier de 0° à 25°, de préférence de 5 à 15°, et/ou sont inclinés respectivement, considéré dans un plan contenant le sens de profondeur et la ligne médiane distale-proximale, par rapport au sens de profondeur selon l'angle aigu, en particulier de 0 à 25°, de préférence de 5 à 20°.

8. Poignée selon l'une quelconque des revendications 3 à 7, **caractérisée en ce que** les sections d'extrémité distales de chaque branche de préhension (13a, 13b) sont configurées ou pourvues de saillies (29c) orientées les unes vers les autres vers l'intérieur, qui sont prévues et configurées afin de servir de limitations de pivotement pour les deux branches de préhension (13a, 13b) **en ce que** celles-ci butent contre la tige d'instrument (7) déjà couplée à l'intérieur du boîtier de préhension (3) central,
dans laquelle de préférence, lors du découplage de la tige d'instrument (7) de la poignée (3), la limitation de pivotement est supprimée et ainsi un pivotement supplémentaire des deux branches de préhension (13a, 13b) est permis dans une position de pivotement de couplage dans laquelle la transmission (9) peut être amenée en et hors prise avec le logement de couplage, de préférence sous la forme d'une fente (39).

9. Poignée selon l'une quelconque des revendications 3 à 8, **caractérisée en ce que** les sections de transmission de force (33a, 33b) sont configurées dans le sens de profondeur au moins d'un côté de manière ouverte, de préférence de manière ouverte vers un côté supérieur de poignée.

10. Poignée selon l'une quelconque des revendications 1 à 9, **caractérisée en ce que** les branches de préhension (13a, 13b) présentent respectivement un œillet pour doigt (13c) qui est configuré ouvert du côté circonférentiel au niveau de ses sections d'œillet proximales respectives, en particulier présentent sensiblement une forme en U ouverte vers la position proximale.

11. Poignée selon l'une quelconque des revendications 1 à 10, **caractérisée en ce que** l'appareil de couplage et de rotation (5) présente une section de couplage de tige pour le couplage au choix, en particulier pour le logement axial/fixe en translation et rotatif de la tige d'instrument (7) au niveau du boîtier de préhension (3) et une section de couplage de transmission pour le couplage au choix, en particulier le logement axial/fixe en translation, de la transmission (9) avec l'engrenage (25).

12. Poignée selon l'une quelconque des revendications 1 à 11, **caractérisée en ce que** les branches de préhension (13a, 13b) sont articulées de manière pivotante au boîtier de préhension (3) respectivement par rapport à une autre branche de préhension des deux branches de préhension (13a, 13b), et l'engrenage (25) présente une section de couplage de branche de préhension couplée ou pouvant être couplée aux branches de préhension (13a, 13b) et est configurée afin de transmettre un mouvement de pivotement des branches de préhension (13a, 13b) dans un mouvement de translation de la transmission (9) en particulier dans l'état de la tige d'instrument (7) couplé à la poignée et à la transmission (9).

13. Poignée d'un ou pour un instrument chirurgical, en particulier un instrument électrochirurgical à tige mini-invasive, avec
- un boîtier de préhension (3) central qui s'étend d'un point distal à proximal ;
- un engrenage (25) logé dans le boîtier de préhension (3) central,
- un dispositif de couplage et de rotation (5) combiné au niveau de la section d'extrémité distale du boîtier de préhension (3) central qui est prévu et configuré pour le couplage au choix aussi bien d'une tige d'instrument (7) au niveau du boîtier de préhension (3) central que d'une transmission (9) logée dans la tige d'instrument (7) au niveau de l'engrenage (25),
- deux branches de préhension (13a, 13b) positionnées diamétralement à l'opposé au niveau du boîtier de préhension (3) qui s'étendent de part et d'autre du boîtier de préhension (3) central d'une position distale dans la direction proximale et qui sont articulées au niveau de leurs sections d'extrémité distales au niveau du boîtier de préhension (3) central de telle manière qu'elles soient mobiles l'une vers l'autre et loin l'une de l'autre,
- des éléments de transmission de force ou des sections de transmission de force (33a, 33b, 41) qui sont configurées ou agencées au niveau de la section d'extrémité distale de chaque branche de préhension (13a, 13b) et sont en prise active avec l'engrenage (25) afin de transmettre le mouvement de branche de préhension à la transmission (9),
deux axes de pivotement (27a, 27b) agencés de manière diamétralement opposée au niveau/dans le boîtier de préhension (3) central, auxquels les branches de préhension (13a, 13b) sont articulées,
dans lequel les sections de transmission de force (41) sont configurées sous la forme de roues dentées ou roues dentées à cercle partiel qui sont en prise d'engrènement avec une roue dentée centrale (43) dont l'axe de rotation (43a) est orienté parallèlement à une ligne imaginaire reliant les axes de pivotement (27a, 27b) et qui présente un logement de couplage, de préférence sous la forme d'une fente (39) pour la transmission (9).

14. Instrument chirurgical, en particulier instrument électrochirurgical à tige mini-invasive avec une tige d'instrument (7), dans laquelle une transmission (9) est de préférence logée sous la forme d'une barre de pression-traction et à la section d'extrémité distale de laquelle un effecteur (11) est agencé, lequel peut être actionné mécaniquement par le biais de la transmission (9) et peut être alimenté en un courant électrique le cas échéant par le biais de conducteurs électriques, **caractérisé par** une poignée (3) selon l'une quelconque des revendications 1 à 13, dans lequel la tige d'instrument (7) et la transmission (9) logée à l'intérieur est ou peut être couplée avec la poignée, en particulier la tige d'instrument (7) est ou peut être couplée au boîtier de préhension (3) central et la transmission (9) est ou peut être couplée à l'engrenage (25) logé dans le boîtier de préhension (3) central.
